# EUROPEAN PATENT APPLICATION

(11) **EP 4 697 375 A1**
(43) Date of publication of application: **18.02.2026**
(21) Application number: 24788959.5
(22) Date of filing: 05.04.2024
(51) Int. Cl.: H01J 37/32, A61C 8/00, A61C 19/02, H05H 1/24, H05H 7/04

(54) **PLASMA TREATMENT VESSEL AND PLASMA TREATMENT DEVICE**

(30) Priority: 14.04.2023 KR 20230049407; 25.05.2023 KR 20230067927; 30.08.2023 KR 20230114881
(71) Applicant: Plasmapp Co., Ltd., Yuseong-gu Daejeon 34141 (KR)
(72) Inventor: LIM, You Bong, Daejeon 34141 (KR)
(74) Representative: Beck & Rössig European Patent Attorneys
(86) International application number: PCT/KR2024/004501
(87) International publication number: WO 2024/215016

(57) **Abstract**

The present invention provides a plasma treatment vessel and a plasma treatment device. The plasma treatment vessel may comprise: a housing having an interior space for accommodating a workpiece receiving container; a connection electrode disposed at one end of the housing and having a portion exposed to the interior space; and a magnetic field forming unit disposed in the housing and forming a magnetic field in the interior space.

## Description

### [Technical Field]

The present invention relates to a plasma treatment vessel and a plasma treatment device.

### [Background Art]

Implants are replacements that restore lost human tissue, but in dentistry, they refer to artificial teeth.

A dental implant is an artificial tooth structure or dental procedure that is formed by implanting an artificial tooth root made of titanium, which is not rejected by the human body, into the bone where a tooth has fallen out to replace the root of a lost tooth, then attaching it to the alveolar bone and fixing a prosthesis to the artificial tooth root.

Typically, a dental implant is made of titanium and includes a fixture that is implanted into the alveolar bone, an abutment that is fixed to the fixture and supports a prosthesis, an abutment screw that fixes the abutment to the fixture, and a prosthesis that is an artificial tooth fixed to the abutment.

Since each component of the dental implant is inserted into human tissue, it is essential to maintain sterility and surface activation, and thus, preventing contamination or damage during packaging, transportation, and opening of the packaging is a crucial task.

In particular, in the case of a specific ampoule for storing the fixture for implant, the fixture is directly implanted into the alveolar bone, and therefore, when the fixture is stored in the ampoule, a configuration is required to maintain sterility of the fixture and prevent contamination and damage.

Meanwhile, with the recent development of the medical industry, plasma treatment is being used in various ways, and in particular, plasma treatment is being used to increase biocompatibility in the transplantation of biomaterials such as implants.

In the case of conventional plasma treatment, there is a problem that plasma is generated locally near the treatment target, making it difficult to treat the surface uniformly, and the treatment target may be damaged and its performance may be degraded as high energy is transmitted to some areas. In addition, when implants are used as treatment targets, there is a problem in ensuring sterility because the sterile packaging should be removed for surface treatment and a specific electrode connection should be connected.

### [Detailed Description of Invention]

### [Technical Problem]

The present invention is directed to providing a plasma treatment vessel and a plasma treatment device that include a magnetic field forming unit capable of rotating plasma to perform uniform plasma treatment on a surface of a workpiece.

### [Technical Solution]

According to one aspect of the present invention, a plasma treatment vessel includes a housing in which an internal space configured to accommodate a workpiece receiving container is formed, a connection electrode disposed at one end of the housing, a portion of which is exposed through the internal space, and a magnetic field forming unit disposed in the housing and configured to form a magnetic field in the internal space.

### [Advantageous Effects]

A plasma treatment vessel according to the present invention includes a magnetic field forming unit so that plasma surface treatment can be performed uniformly on a workpiece while plasma formed in an internal space rotates.

The plasma treatment vessel according to the present invention is provided with a protrusion on a connection electrode so that a workpiece receiving container can be stably seated. In addition, since the connection electrode can be formed closer to the workpiece due to the protrusion, more efficient plasma surface treatment can be performed.

The plasma treatment vessel according to the present invention can perform plasma surface treatment while the workpiece receiving container is directly accommodated in the internal space so that there is no need to separate the workpiece, such as a fixture of an implant, from a receiving container for surface treatment and put the workpiece into a separate container or connect the workpiece to a support. In this way, convenience of use can be secured and the workpiece can be prevented from being contaminated again after surface treatment.

The plasma treatment vessel according to the present invention can efficiently perform plasma surface treatment while minimizing damage to the workpiece by controlling the density of plasma formed in the internal space by an auxiliary electrode.

In the plasma treatment vessel according to the present invention, the internal space capable of accommodating the workpiece can be formed with the connection electrode and the auxiliary electrode, the magnetic field forming unit can be disposed in contact with the auxiliary electrode to form plasma more intensively around the workpiece, and the formed plasma can rotate to efficiently perform plasma surface treatment.

The plasma treatment vessel according to the present invention can improve the uniformity of plasma surface treatment while maintaining the sterility of the workpiece and can be easily compatible with various types of workpiece receiving containers.

### [Description of Drawings]

FIG. 1 is a perspective view illustrating a plasma treatment vessel according to one embodiment of the present invention.
FIG. 2 is a cross-sectional view illustrating a state in which the plasma treatment vessel of FIG. 1 accommodates a workpiece receiving container.
FIG. 3 is a cross-sectional view illustrating a modified example of the plasma treatment vessel of FIG. 2.
FIG. 4 is a cross-sectional view along line IV-IV of FIG. 1.
FIGS. 5 and 6 are diagrams illustrating another embodiment of the magnetic field forming unit of FIG. 1.
FIG. 7 is a cross-sectional view illustrating a plasma treatment vessel according to another embodiment of the present invention.
FIG. 8 is a perspective view illustrating a plasma treatment device according to one embodiment of the present invention.
FIG. 9 is a conceptual diagram illustrating a state in which the plasma treatment device of FIG. 8 performs plasma surface treatment on a workpiece.
FIG. 10 to 15 are conceptual diagrams illustrating a state in which various modified examples of plasma treatment vessels are mounted on the plasma treatment device.
FIG. 16 is a conceptual diagram illustrating a state in which a plasma treatment device according to another embodiment of the present invention performs plasma surface treatment on a workpiece.
FIGS. 17 and 18 are diagrams illustrating modified examples of the plasma treatment device of FIG. 16.

### [Best Mode]

According to one aspect of the present invention, there is provided a plasma treatment vessel including a housing in which an internal space is formed to accommodate a workpiece receiving container, a connection electrode disposed at one end of the housing, a portion of which is exposed through the internal space, and a magnetic field forming unit disposed in the housing and configured to form a magnetic field in the internal space.

In addition, the magnetic field forming unit may be disposed symmetrically based on the internal space.

In addition, the magnetic field forming unit may include a ring-shaped magnet surrounding the internal space.

In addition, the magnetic field forming unit may include a plurality of magnets disposed to be spaced apart from and face each other.

In addition, the magnetic field forming unit may form the magnetic field to allow plasma formed in the internal space to rotate based on the internal space.

In addition, the magnetic field forming unit may form the magnetic field in a direction intersecting a direction of an electric field formed by the connection electrode.

In addition, the magnetic field forming unit may be disposed adjacent to a workpiece accommodated in the workpiece receiving container.

In addition, the magnetic field forming unit may be disposed adjacent to the connection electrode.

In addition, the magnetic field forming unit may include two or more magnetic field forming units.

In addition, at least a portion of the housing may be made of a transparent material such that plasma treatment is checked from the outside.

In addition, the connection electrode may include a protrusion which protrudes toward the internal space and on which the workpiece receiving container may be seated.

In addition, the plasma treatment vessel may further include an auxiliary electrode disposed in the housing to surround the workpiece receiving container and disposed adjacent to the workpiece accommodated in the workpiece receiving container.

In addition, the auxiliary electrode may be electrically connected to the connection electrode, and the magnetic field forming unit may be disposed in contact with the auxiliary electrode.

In addition, the magnetic field forming unit may be disposed between the auxiliary electrode and the connection electrode.

According to another aspect of the present disclosure, there is provided a plasma treatment device including a sealing part of which an interior accommodates a plasma treatment vessel, wherein the interior is sealed from the external environment, an exhaust part configured to exhaust the atmosphere inside the sealing part and form a low-pressure atmosphere within a preset process pressure range inside the sealing part, and a treatment part configured to form an electric field inside the sealing part and discharge the low-pressure atmosphere, wherein the plasma treatment vessel includes a housing in which an internal space is formed to accommodate a workpiece receiving container, a connection electrode disposed at one end of the housing, a portion of which is exposed through the internal space, and a magnetic field forming unit disposed in the housing and configured to form a magnetic field in the internal space.

In addition, the treatment part may include a first electrode electrically connected to the connection electrode and a second electrode disposed to be spaced apart from the first electrode.

In addition, the plasma treatment vessel may further include a coupling magnet that fixes the connection electrode to the first electrode.

In addition, the magnetic field forming unit may be disposed symmetrically based on the internal space.

In addition, the magnetic field forming unit may form the magnetic field in a direction intersecting a direction of an electric field formed by the connection electrode.

In addition, the magnetic field forming unit may be disposed adjacent to a workpiece accommodated in the workpiece receiving container.

In addition, the magnetic field forming unit may be disposed adjacent to the connection electrode.

In addition, the connection electrode may include a protrusion which protrudes toward the internal space and on which the workpiece receiving container may be seated.

In addition, the plasma treatment vessel may further include an auxiliary electrode disposed in the housing to surround the workpiece receiving container and disposed adjacent to the workpiece accommodated in the workpiece receiving container.

In addition, the auxiliary electrode may be electrically connected to the connection electrode, and the magnetic field forming unit may be disposed in contact with the auxiliary electrode.

In addition, the magnetic field forming unit may be disposed between the auxiliary electrode and the connection electrode.

According to still another aspect of the present disclosure, there is provided a plasma treatment device including a sealing part of which an interior accommodates a workpiece receiving container, wherein the interior is sealed from the external environment, an exhaust part configured to exhaust the atmosphere inside the sealing part and exhaust a low-pressure atmosphere within a preset process pressure range in the interior of the sealing part, and a treatment part configured to form an electric field inside the sealing part and discharge the low-pressure atmosphere, and a magnetic field forming unit configured to form a magnetic field in the interior of the sealing part, wherein the magnetic field forming unit rotates the discharged atmosphere.

In addition, the treatment part may include a first electrode electrically connected to the workpiece receiving container and a second electrode disposed to be spaced apart from the first electrode.

In addition, the magnetic field forming unit may be disposed adjacent to one of the first electrode and the second electrode.

In addition, the magnetic field forming unit may be disposed symmetrically based on the interior of the sealing part.

In addition, the magnetic field forming unit may be disposed adjacent to a workpiece accommodated in the workpiece receiving container.

In addition, the treatment part may further include an auxiliary electrode disposed in the housing to surround the workpiece receiving container and disposed adjacent to the workpiece accommodated in the workpiece receiving container.

In addition, the magnetic field forming unit may be disposed between the auxiliary electrode and the second electrode.

According to yet another aspect of the present disclosure, there is provided a plasma treatment vessel including a connection electrode having an internal space configured to accommodate a workpiece receiving container, and a magnetic field forming unit disposed in contact with the connection electrode and configured to form a magnetic field in the internal space.

In addition, the magnetic field forming unit may be disposed to be located inside the connection electrode, and the connection electrode may include an opening through which the workpiece receiving container may be inserted or removed.

In addition, the connection electrode may include a protrusion which protrudes toward the internal space and on which the workpiece receiving container may be seated.

In addition, the plasma treatment vessel may further include an auxiliary electrode disposed to surround the workpiece receiving container.

In addition, the connection electrode and the auxiliary electrode may be formed integrally.

### [Modes of the Invention]

Hereinafter, the configuration and operation of the present invention will be described in detail with reference to embodiments of the present invention shown in the accompanying drawings.

The present invention may be modified into various forms and may have a variety of exemplary embodiments, and therefore, specific embodiments will be illustrated in the accompanying drawings and described in detail. Effects and features of the present invention and methods for achieving them will be made clear from embodiments described in detail below with reference to the accompanying drawings. However, the present invention is not limited to the exemplary embodiments described below and may be implemented in various forms.

Hereinafter, preferred embodiments of the present invention will be described in detail with reference to the drawings, and in describing with reference to the accompanying drawings, the same or corresponding components are assigned the same reference numerals, and duplicate descriptions thereof will be omitted therein.

In the following embodiments, the singular form includes the plural form unless the context clearly notes otherwise.

In the embodiments, terms such as "include" or "have" mean that a feature or component described in the specification is present, and do not preclude the possibility that one or more other features or components may be added.

In some embodiments, when other implementations are possible, specific process sequences may be performed in a different order than described. For example, two processes described consecutively may be performed substantially simultaneously or may proceed in the reverse order from that described.

For convenience of description, in the drawings, sizes of components may be exaggerated or reduced. For example, a size and a thickness of each component shown in the drawings are arbitrarily illustrated for convenience of description, and thus the embodiments below are not necessarily limited to those shown in the drawings.

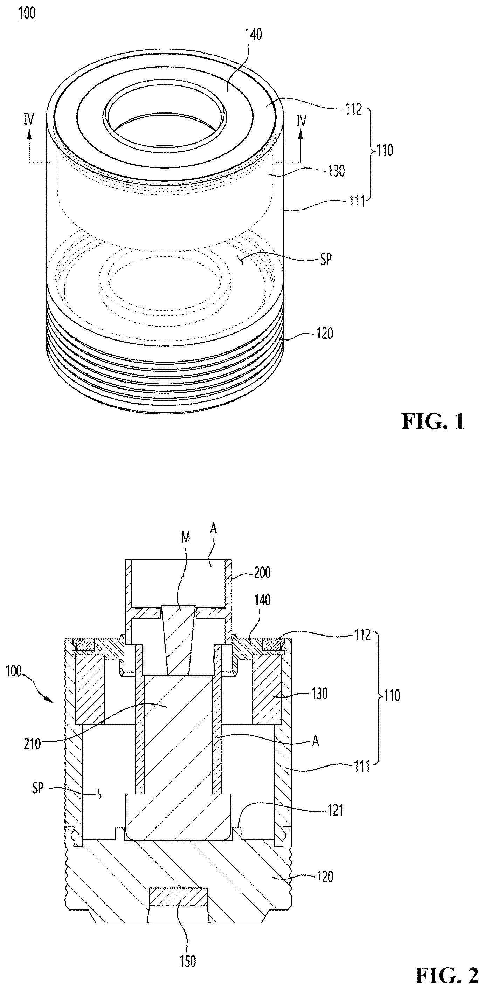

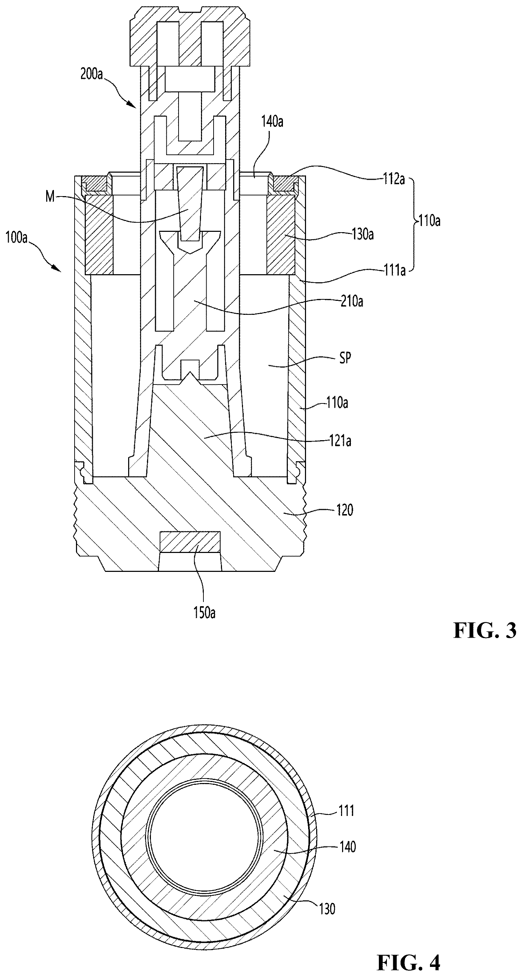

FIG. 1 is a perspective view illustrating a plasma treatment vessel according to one embodiment of the present invention, and FIG. 2 is a cross-sectional view illustrating a state in which the plasma treatment vessel of FIG. 1 accommodates a workpiece receiving container 200. In addition, FIG. 3 is a cross-sectional view illustrating a modified example of the plasma treatment vessel of FIG. 2, FIG. 4 is a cross-sectional view along line IV-IV of FIG. 1, and FIGS. 5 and 6 are diagrams illustrating another embodiment of the plasma treatment device of FIG. 1.

Hereinafter, a "workpiece M" refers to a target on which plasma surface treatment is performed. For example, the "workpiece M" may be a dental implant. However, the present invention is not limited thereto, and any target to which characteristics are imparted according to plasma treatment may be the target and may be selected from the group including dental implants, orthopedic implants, bone grafts, skin grafts, ophthalmic implants, cardiac implants, cochlear implants, cosmetic implants, neural implants, medical resins, dental prostheses, fibers, seeds, and foods.

The plasma treatment vessel 100 may accommodate the workpiece receiving container 200 that receives the workpiece M. The plasma treatment vessel 100 may be formed in a holder shape that accommodates at least a portion of the workpiece receiving container 200, and in this case, a portion of the workpiece receiving container 200 may be exposed to the outside. The plasma treatment vessel 100 has an opening formed corresponding to a shape of the workpiece receiving container 200 and may accommodate the workpiece receiving container 200 through the opening. The plasma treatment vessel 100 is mounted on a plasma treatment device 10 which will be described below, and thus plasma surface treatment of the workpiece M may be performed inside the plasma treatment vessel 100.

Referring to FIGS. 1 and 2, the plasma treatment vessel 100 according to one embodiment of the present invention may include a housing 110, a connection electrode 120, and a magnetic field forming unit 130.

The housing 110 forms an exterior of the plasma treatment vessel 100 and may have an internal space SP. The workpiece receiving container 200 may be accommodated in the internal space SP of the housing 110.

In one embodiment, the housing 110 may have a side wall 111 and an upper cover 112. For example, as shown in FIG. 1, the side wall 111 may have a cylindrical structure and the upper cover 112 may be a circular plate shape corresponding to the side wall 111. However, the present invention is not limited thereto and, of course, the side wall 111 and the upper cover 112 may be formed in various shapes to be mounted on the plasma treatment device 10.

The upper cover 112 may have an opening corresponding to a cross-sectional shape of the workpiece receiving container 200, and the workpiece receiving container 200 may be inserted through the opening and accommodated in the internal space SP.

In one embodiment, the housing 110 may be made of a non-conductive material. For example, the housing 110 may be made of a resin such as polypropylene (PP), polycarbonate (PC), polyacetal (POM), polystyrene, polyamide, polyethylene, rigid polyvinyl chloride, or an acrylonitrile-butadiene-styrene copolymer (ABS).

In one embodiment, at least a portion of the housing 110 may be made of a transparent material. In this way, a user may visually observe the surface treatment process of the workpiece M inside the plasma treatment device 10.

The connection electrode 120 may be disposed at one end of the housing 110. In one embodiment, the connection electrode 120 may be disposed at a lower end of the housing 110 and coupled to a lower block 12 of the plasma treatment device 10 which will be described below. In this case, the connection electrode 120 may be electrically connected to a first electrode E1 of the plasma treatment device 10.

A portion of the connection electrode 120 may be exposed through the internal space SP. The connection electrode 120 may be connected to an external power source to form an electric field in the internal space SP, thereby generating plasma in the internal space SP.

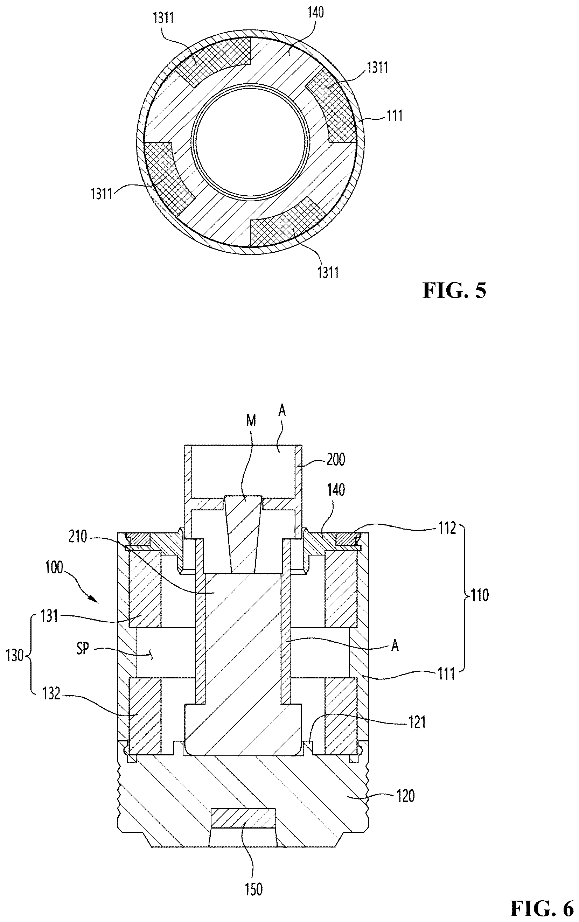

In one embodiment, the connection electrode 120 may include an auxiliary electrode 140 that extends outside the internal space SP and is formed to surround the workpiece receiving container 200. The auxiliary electrode 140 is a component distinct from the protrusion 121, which will be described below, and may extend until it comes into contact with the magnetic field forming unit 130 and may be formed to surround the magnetic field forming unit 130. Meanwhile, as in one example shown in FIG. 6, when the magnetic field forming unit 130 is disposed in direct contact with the connection electrode 120, the connection electrode 120 may not be provided with the auxiliary electrode 140.

In one embodiment, the connection electrode 120 may be provided with the protrusion 121 protruding toward the internal space SP. A portion of the workpiece receiving container 200 is coupled to the protrusion 121 so that the workpiece receiving container 200 may be stably seated in the plasma treatment vessel 100.

In one embodiment, the connection electrode 120 may include an electrode cover (not shown). The electrode cover may be made of a non-conductive material and may cover a portion of the connection electrode 120. In this way, the electrode cover may prevent plasma from being generated near the connection electrode 120, thereby controlling the plasma to be generated intensively in the workpiece receiving container 200.

A shape of the protrusion 121 may be formed in various ways according to the type of workpiece receiving container 200. For example, as shown in FIG. 2, when the workpiece receiving container 200 has a general cylindrical shape, the protrusion 121 may be formed to surround a lower end of the workpiece receiving container 200.

Alternatively, as shown in FIG. 3, a protrusion 121a may be formed in various structures according to the shape of a workpiece receiving container 200a. For example, in the case of a structure in which one region of a lower portion of the workpiece receiving container 200a is open, the protrusion 121a may be formed to correspond to the open region. In this case, the protrusion 121a may extend toward a support block 210a supporting the workpiece M to come into contact with the support block 210a.

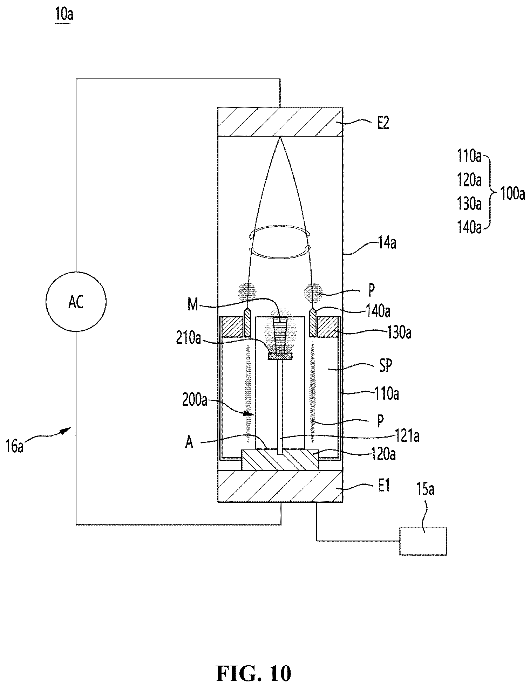

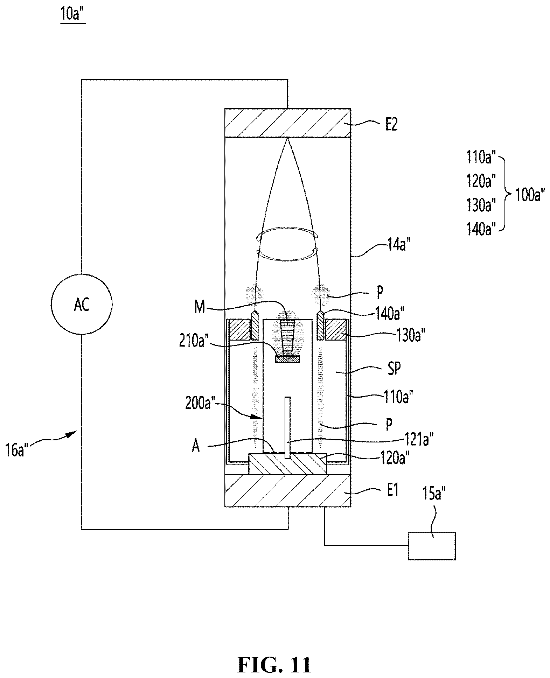

In another embodiment, as shown in FIG. 10, the protrusion 121a may be formed to be in direct contact with the workpiece M such that the workpiece is electrically connected to a connection electrode 120a. Alternatively, as shown in FIG. 11, a protrusion 121a" may be formed adjacent to the workpiece M without being in direct contact therewith. Meanwhile, when the workpiece M is a conductor such as a dental implant fixture, the protrusion 121a may extend adjacent to the workpiece M so that the workpiece M acts like a floating electrode, thereby increasing the efficiency of surface treatment while minimizing surface damage to the workpiece M.

The magnetic field forming unit 130 may form a magnetic field in the internal space SP. When plasma is formed in the internal space SP by the connection electrode 120, there may be a case in which the plasma is unintentionally non-uniformly formed. According to the present invention, a magnetic field may be formed by including the magnetic field forming unit 130 to rotate the plasma formed in the internal space SP.

That is, the magnetic field forming unit 130 may provide a magnetic field that rotates the plasma formed in the internal space SP, and the plasma (or plasma constituent particles) may rotate (spiral) around the workpiece M due to the magnetic field generated by the magnetic field forming unit 130, thereby uniformly treating a surface of the workpiece M with the plasma. In this case, the plasma may perform surface treatment on the workpiece M inside the workpiece receiving container 200 through the open area of the workpiece receiving container 200.

Specifically, the magnetic field forming unit 130 may form a magnetic field in a direction intersecting the direction of the electric field formed in the internal space SP by the connection electrode 120. For example, the magnetic field forming unit 130 may form a magnetic field in a direction perpendicular to the direction of the electric field formed in the internal space SP. Here, the magnetic field formed by the magnetic field forming unit 130 does not need to be entirely perpendicular to the electric field direction, and at least a portion of the magnetic field may be formed perpendicular to the electric field direction. Therefore, the magnetic field forming unit 130 may rotate the plasma generated in the internal space SP.

In one embodiment, the magnetic field forming unit 130 may include a permanent magnet. Alternatively, the magnetic field forming unit 130 may include an electromagnet connected to an external power source.

The magnetic field forming unit 130 may be disposed in the housing 110. In this case, the magnetic field forming unit 130 may be disposed symmetrically based on the internal space SP.

In one embodiment, the magnetic field forming unit 130 may be a single magnet surrounding the internal space SP. FIG. 4 shows an embodiment in which the magnetic field forming unit 130 is a ring-shaped magnet surrounding the internal space SP.

In another embodiment, the magnetic field forming unit 130 may include a plurality of magnets 1311 disposed to be spaced apart from and face each other based on the internal space SP. FIG. 5 shows illustrates an embodiment in which the magnetic field forming unit 130 includes four magnets disposed to be spaced apart from and face each other.

In one embodiment, the magnetic field forming unit 130 may be disposed adjacent to a workpiece M accommodated in the workpiece receiving container 200. For example, as shown in FIG. 2, the magnetic field forming unit 130 may be disposed adjacent to the workpiece M below the upper cover 112.

In another embodiment, the magnetic field forming unit 130 may be disposed adjacent to the connection electrode 120. The arrangement of the magnetic field forming unit 130 is not limited thereto and may be disposed at various locations inside or outside the housing 110.

In still another embodiment, the magnetic field forming unit 130 may be disposed between the auxiliary electrode 140, which will be described below, and the connection electrode 120. The density of the plasma may be controlled by the auxiliary electrode 140. In this case, the magnetic field forming unit 130 may be disposed between the auxiliary electrode 140 and the connection electrode 120 to uniformly rotate the plasma.

In one embodiment, the magnetic field forming unit 130 may be provided as two or more magnetic field forming units. The magnetic field forming unit may include the ring magnet or the plurality of magnets described above.

For example, as shown in FIG. 6, the magnetic field forming unit 130 may include two magnetic field forming units 131 and 132 disposed adjacent to the upper cover 112 and the connection electrode 120, respectively. Alternatively, the magnetic field forming unit 130 may include a plurality of magnetic field forming units stacked above the connection electrode 120.

However, hereinafter, for convenience of description, the description will focus on an example in which the magnetic field forming unit 130 has a single ring-shaped permanent magnet disposed adjacent to the upper cover 112.

The plasma treatment vessel 100 according to one embodiment of the present invention may further include the auxiliary electrode 140 and a coupling magnet 150.

The auxiliary electrode 140 may be disposed in the housing 110 to surround the workpiece receiving container 200. The auxiliary electrode 140 may be disposed to be spaced apart from the connection electrode 120 to control the electric field formed in the internal space SP and the density of plasma generated according to the electric field.

In one embodiment, the auxiliary electrode 140 may be a floating electrode to which no external potential is directly applied. A potential of the floating electrode may be set differently according to relative distances from the first electrode E1 and the second electrode E2, which will be described below. In this case, physical properties of the workpiece M may be taken into consideration.

In one embodiment, the auxiliary electrode 140 may be disposed adjacent to the workpiece M accommodated in the workpiece receiving container 200. Plasma may be intensively generated and rotated in an area adjacent to the workpiece M by the auxiliary electrode 140. In this way, the plasma treatment vessel 100 may increase the efficiency of plasma surface treatment.

For example, as shown in FIG. 2, the auxiliary electrode 140 may be disposed between the upper cover 112 and the magnetic field forming unit 130 to be adjacent to the workpiece M. In this case, the auxiliary electrode 140 may form the internal space SP of the housing 110 together with the upper cover 112.

The coupling magnet 150 may couple the connection electrode 120 to the lower block 12 of the plasma treatment device 10. The coupling magnet 150 is in contact with the fixed magnet of the lower block 12 so that the plasma treatment vessel 100 may be stably seated on the plasma treatment device 10 due to a magnetic force.

The coupling magnet 150 may be disposed at various locations where the plasma treatment vessel 100 is in contact with the plasma treatment device 10. In one embodiment, as shown in FIG. 2, the coupling magnet 150 may be disposed in a lower central region of the connection electrode 120.

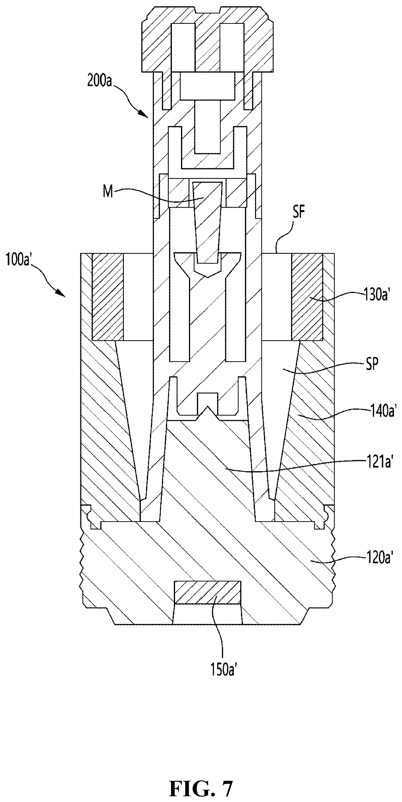

FIG. 7 is a cross-sectional view illustrating a plasma treatment vessel 100a' according to another embodiment of the present invention.

Referring to FIG. 7, the plasma treatment vessel 100a' according to another embodiment of the present invention may be provided with a connection electrode 120a' and a magnetic field forming unit 130a'.

The connection electrode 120a' may accommodate a workpiece receiving container 200a. Specifically, the connection electrode 120a' may have an internal space for accommodating the workpiece receiving container 200a. In addition, the connection electrode 120a may have an opening SF capable of inserting or removing the workpiece receiving container 200a. The workpiece receiving container 200a may be disposed in the internal space SP through the opening SF.

The connection electrode 120a' may protrude toward the internal space SP and have a protrusion 121a' on which the workpiece receiving container 200a may be seated.

In one embodiment, the protrusion 121a' may be formed by extending a portion of the connection electrode 120a' toward the internal space SP. In this case, the workpiece receiving container 200a may be stably seated on the connection electrode 120a' by the protrusion 121a'. In addition, since the protrusion 121a' is formed integrally with the connection electrode 120a', plasma may be formed intensively on the workpiece M.

The magnetic field forming unit 130a' may be disposed in contact with the connection electrode 120a' (which refers to the entire connection electrode formed integrally with the auxiliary electrode 140a' in FIG. 7). In this case, the magnetic field forming unit 130a' may form a magnetic field in the internal space SP of the connection electrode 120a' to rotate the plasma generated in the internal space SP.

In one embodiment, as shown in FIG. 7, the magnetic field forming unit 130a' may be disposed to be located inside the connection electrode 120a' (which refers to the entire connection electrode formed integrally with the auxiliary electrode 140a' in FIG. 7). Alternatively, the magnetic field forming unit 130a' may be disposed in contact with the connection electrode 120a' (which refers to the entire connection electrode formed integrally with the auxiliary electrode 140a' in FIG. 7).

Meanwhile, unlike the embodiment of FIG. 7, in a case in which the auxiliary electrode is not formed integrally with the connection electrode (not shown), the magnetic field forming unit may be disposed in contact with only the auxiliary electrode and may form a magnetic field in an internal space formed by the auxiliary electrode and the connection electrode.

Meanwhile, for the specific configuration and function of the magnetic field forming unit 130a', refer to the content described above.

The plasma treatment vessel 100a' may further include an auxiliary electrode 140a'. Plasma may be intensively generated and rotated in an area adjacent to the workpiece M by the auxiliary electrode 140a'. In this way, the plasma treatment vessel 100a' may increase the efficiency of plasma surface treatment.

The auxiliary electrode 140a' may be disposed to surround the workpiece receiving container 200a. FIG. 7 shows an embodiment in which the auxiliary electrode 140a' is formed integrally with the connection electrode 120a'. However, the arrangement and shape of the auxiliary electrode 140a' are not limited thereto, and the auxiliary electrode 140a' may be disposed on an inner wall of the container-shaped connection electrode 120a' to doubly surround the workpiece receiving container 200a together with the connection electrode 120a'.

In one embodiment, the auxiliary electrode 140a' may be formed integrally with the connection electrode 120a' to have a side wall shape that forms the internal space SP. FIG. 7 shows an embodiment in which the auxiliary electrode 140a' has a side wall shape with an inclined inner wall, but the shape of the auxiliary electrode 140a' is not limited thereto.

As described above, the auxiliary electrode 140a' may be formed integrally with the connection electrode 120a' and electrically connected thereto. Alternatively, the auxiliary electrode 140a' may be a floating electrode having a separate potential from the connection electrode 120a'. The electric field formed in the internal space SP by the auxiliary electrode 140a' and the density of plasma generated by the electric field may be controlled.

The plasma treatment vessel 100a' according to another embodiment of the present invention may further include a coupling magnet 150a'. In addition, the plasma treatment vessel 100a' may be mounted on the plasma treatment device 10 which will be described below. In this regard, reference will be made to previously described content.

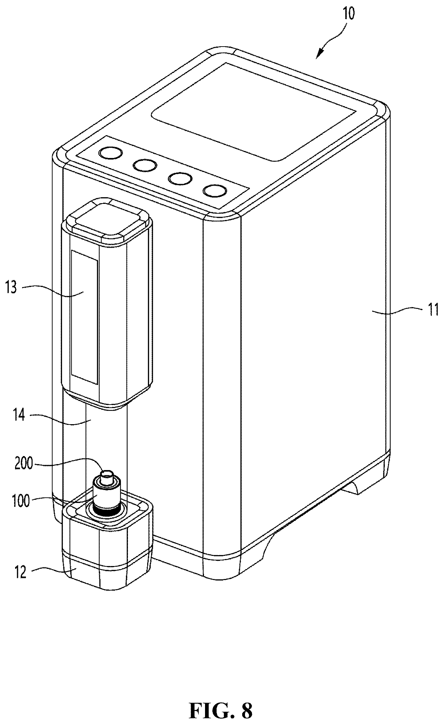

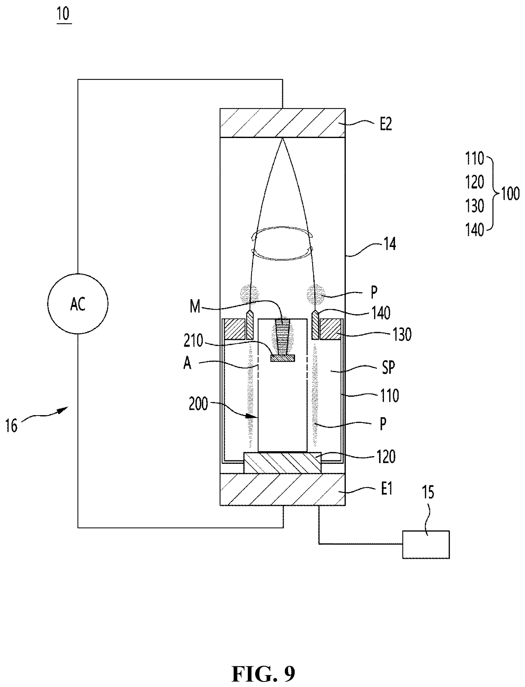

FIG. 8 is a perspective view illustrating the plasma treatment vessel 10 according to one embodiment of the present invention, and FIG. 9 is a conceptual diagram illustrating a state in which the plasma treatment device 10 of FIG. 8 performs plasma surface treatment on the workpiece M.

Referring to FIGS. 8 and 9, the plasma treatment device 10 may include a body 11, the lower block 12, an upper block 13, a sealing part 14, an exhaust part 15, and a treatment part 16. The plasma treatment device 10 may accommodate the plasma treatment vessel 100 and perform plasma surface treatment on the workpiece M disposed inside the plasma treatment vessel 100.

The body 11 may form an exterior of the plasma treatment device 10. The lower block 12 and the upper block 13 may be disposed on one side of the body 11.

The lower block 12 may be disposed and located on a front side of the body 11. The lower block 12 may be disposed below the upper block 13. A first electrode E1 for supplying power to the plasma treatment device 10 may be disposed on an upper surface of the lower block 12.

A seating portion for accommodating the plasma treatment vessel 100 may be formed on the lower block 12. As described above, the lower block 12 may accommodate various embodiments of plasma treatment vessels 100.

In one embodiment, the lower block 12 may be provided with a fixing magnet. The fixing magnet may be in contact with the coupling magnet 150 of the plasma treatment vessel 100 and stably fix the plasma treatment vessel 100 to the plasma treatment device 10 by a magnetic force.

The upper block 13 may be disposed to be located at the front side of the body 11 and above the lower block 12. The upper block 13 may be provided with a raising/lowering part (not shown) for raising/lowering the sealing part 14.

The sealing part 14 may be moved relative to the lower block 12 to seal the plasma treatment vessel 100 from the external environment. For example, when the sealing part 14 is raised or lowered, a lower portion of the sealing part 14 may come into contact with an upper surface of the lower block 12, thereby forming a sealed space inside the sealing part 14.

The exhaust part 15 may exhaust air inside the sealed sealing part 14 from the external environment, thereby forming a low-pressure atmosphere within a preset process pressure range.

In one embodiment, an exhaust hole connecting the sealing part 14 and the exhaust part 15 may be formed in the lower block 12. The air inside the sealing part 14 may be exhausted through the exhaust hole.

The treatment part 16 may generate plasma P by discharging the low-pressure atmosphere in a hollow interior of the sealing part 14 in which a sealed space is formed and perform plasma treatment on the workpiece M.

The treatment part 16 may include the first electrode E1, the second electrode E2, and a power supply AC.

The first electrode E1 may be disposed in the lower block 12 to be electrically connected to the connection electrode 120 of the plasma treatment vessel 100.

The second electrode E2 may be disposed on an upper portion of the sealing part 14 or the upper block 13 to be disposed on an upper portion of the plasma treatment vessel 100.

The power supply AC may supply power to the first electrode E1 and the second electrode E2. The second electrode E2 is disposed to be spaced apart from the first electrode E1, such that an electric field is formed inside the sealing part 14 by the power supply AC, and the low-pressure atmosphere is discharged so that plasma P may be generated.

Hereinafter, focusing on a relationship between configurations of the plasma treatment vessel 100 and the plasma treatment device 10, a principle by which the plasma treatment device 10 performs plasma surface treatment on the workpiece M will be described.

The workpiece M may be accommodated inside the workpiece receiving container 200. In this case, the workpiece M may be fixed inside the workpiece receiving container 200 by a support block 210. In addition, at least a portion of the workpiece receiving container 200 is open so that the plasma P may be generated directly inside the workpiece receiving container 200 or moved and disposed therein. FIG. 8 shows an embodiment in which a portion of a side surface of the workpiece receiving container 200 is open.

The workpiece receiving container 200 may be accommodated in the internal space SP of the plasma treatment vessel 100. For example, the workpiece receiving container 200 may be seated on the connection electrode 120.

When the power supply AC applies a voltage to the first electrode E1 and the second electrode E2, a potential may be formed at the connection electrode 120 connected to the first electrode E1. In this way, an electric field may be formed inside the sealing part 14, including the internal space SP of the plasma treatment vessel 100.

When the air inside the sealing part 14 is exhausted by the exhaust part 15 to form a low-pressure atmosphere within a preset process pressure range, the atmosphere may be discharged by the electric field to generate the plasma P. As described above, at least a portion of the workpiece receiving container 200 is open so that plasma surface treatment may be performed on the workpiece M.

A potential may also be formed at the auxiliary electrode 140 disposed between the first electrode E1 and the second electrode E2. In this case, the density of the plasma P inside the sealing part 14 may be controlled. The plasma P may be concentrated in an area adjacent to the workpiece M by the auxiliary electrode 140.

The plasma P inside the sealing part 14 may rotate by the magnetic field forming unit 130 of the plasma treatment vessel 100. The magnetic field forming unit 130 forms a magnetic field in a direction intersecting a direction of the electric field so that the plasma P may rotate inside the sealing part 14. The plasma surface treatment may be performed uniformly on a surface of the workpiece M by the rotation of plasma P.

FIG. 10 to 15 are conceptual diagrams illustrating a state in which various modified examples of plasma treatment vessels 100 are mounted on the plasma treatment device 10.

The plasma treatment device 10 may have different configurations of some components of the plasma treatment vessel 100 and a different plasma density inside the sealing part 14 according to a loading method of the workpiece M. Therefore, the following description focuses on these specific configurations, and other configurations and plasma surface treatment methods will be described with reference to the descriptions of FIGS. 8 and 9.

FIG. 10 shows a plasma treatment device 10a provided with the workpiece receiving container 200a and the plasma treatment vessel 100a of FIG. 3.

Referring to FIG. 10, a lower surface of the workpiece receiving container 200a is open so that the protrusion 121a of the connection electrode 120a may be in direct contact with the support block 210a that supports the workpiece M. The connection electrode 120a extends toward the support block 210a to directly apply a potential thereto so that the plasma P may be directly generated inside the workpiece receiving container 200a.

Referring to FIG. 11, a lower surface of the workpiece receiving container 200a" is open so that the protrusion 121a" of the connection electrode 120a" may be disposed adjacent to the support block 210a" that supports the workpiece M. In this case, the protrusion 121a" is not in direct contact with the support block 210a" but may serve as a floating electrode when the workpiece M is a conductor such as a dental implant fixture. Therefore, the efficiency of surface treatment may be increased while minimizing surface damage to the workpiece M.

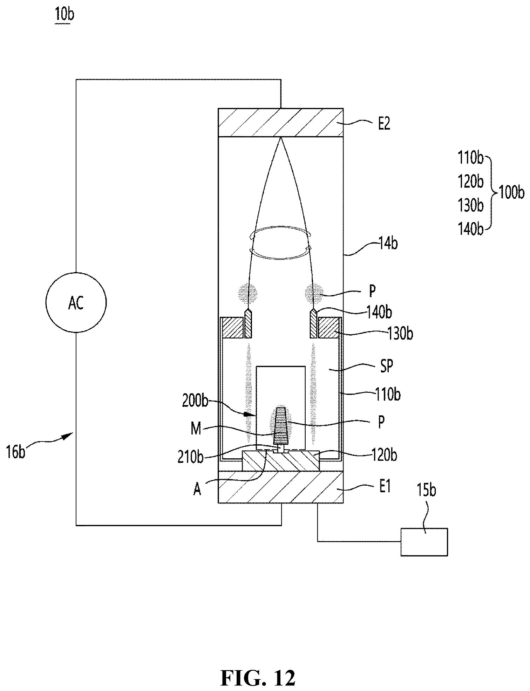

Referring to FIG. 12, the lower surface of the workpiece receiving container 200b is open and the support block 210b may be formed in a pin type. In this case, the pin type support block 210b is in direct contact with the connection electrode 120b and receives a potential, and thus, as shown in FIG. 9, the plasma P may be directly generated inside the workpiece receiving container 200b.

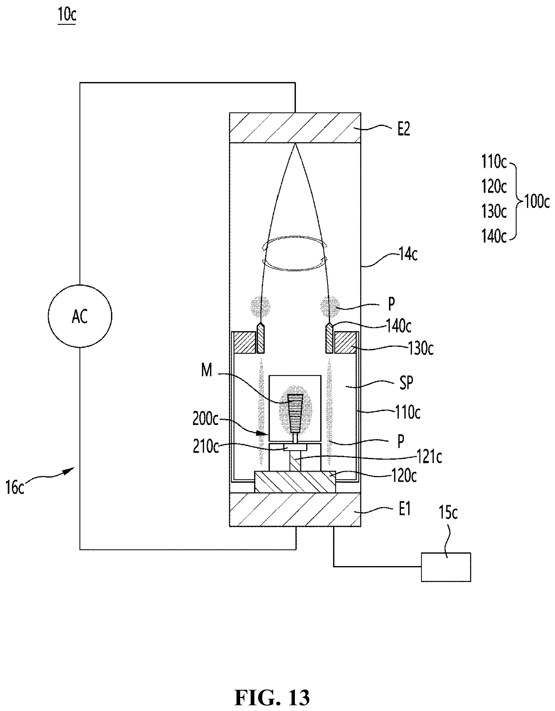

Referring to FIG. 13, the workpiece M and a mount type support block 210c may be disposed in separate spaces in the workpiece receiving container 200c. The support block 210c may be in direct contact with the protrusion 121c of the connection electrode 120c in a lower space, and thus, the plasma P may be generated directly in an upper space in which the workpiece M is disposed.

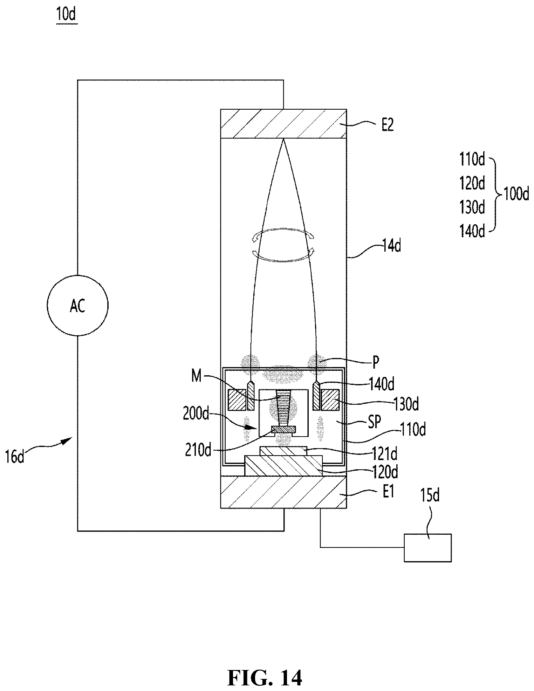

Referring to FIG. 14, a lower portion of the workpiece receiving container 200d may be open so that a support block 210d may be exposed through the internal space SP. In this case, the plasma P is formed between the support block 210d and the connection electrode 120d by the auxiliary electrode 140d, which is a floating electrode, and the connection electrode 120d so that the workpiece M may be directly surface-treated with the plasma P.

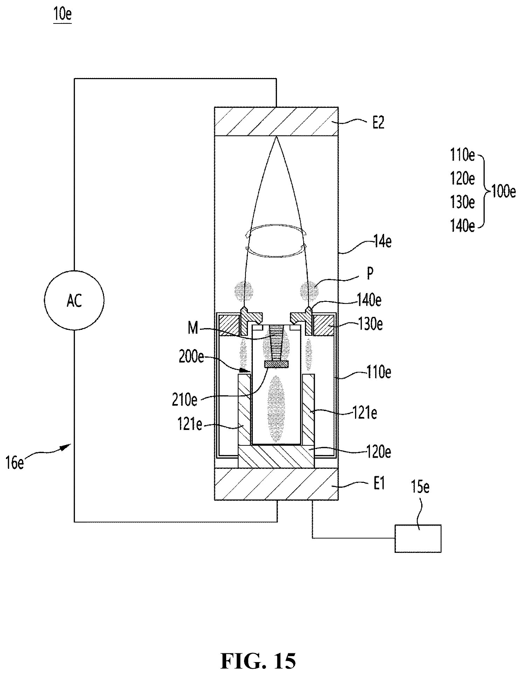

Referring to FIG. 15, a connection electrode 120e of a plasma treatment vessel 100e may be formed to surround a lower portion of a workpiece receiving container 200e. A voltage may be directly applied to the workpiece M by an auxiliary electrode 140e, which is a floating electrode, and a connection electrode 120e, and thus, plasma P is formed inside the workpiece receiving container 200e so that the workpiece M may be surface-treated.

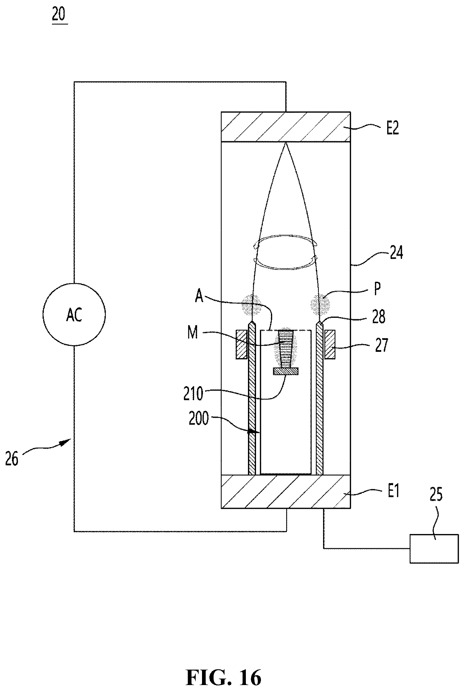

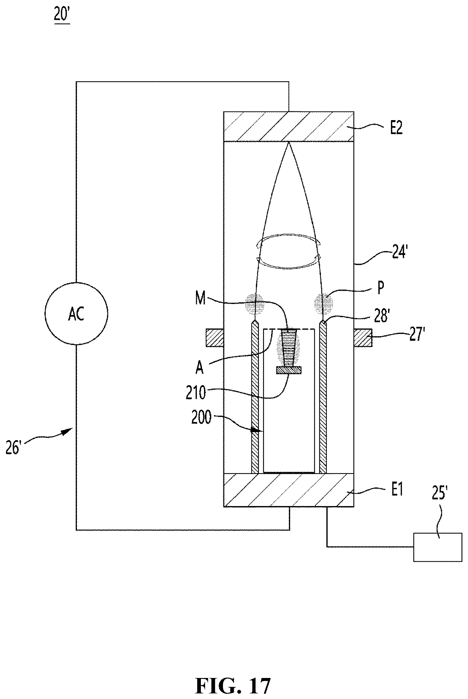

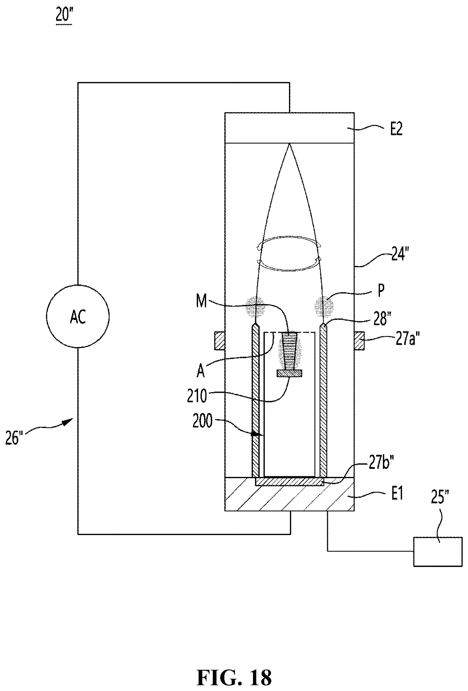

FIG. 16 is a conceptual diagram illustrating a state in which a plasma treatment device 20 according to another embodiment of the present invention performs plasma surface treatment on the workpiece M, and FIGS. 17 and 18 are diagrams illustrating modified examples of the plasma treatment device 20 of FIG. 14.

Referring to FIGS. 16 to 18, the plasma treatment device 20 according to another embodiment of the present invention may include a sealing part 24, an exhaust part 25, a treatment part 26, and a magnetic field forming unit 27.

The workpiece receiving container 200 may be directly accommodated in the sealing part 24 of the plasma treatment device 20 according to another embodiment of the present invention. In this case, the workpiece receiving container 200 may be electrically connected to the first electrode E1.

The magnetic field forming unit 27 may be disposed in the sealing part 24 and form a magnetic field inside the sealing part 24. In this way, the magnetic field forming unit 27 may rotate a discharged atmosphere inside the sealing part 24 by the treatment part 26.

The magnetic field forming unit 27 may be disposed symmetrically based on an interior of the sealing part 24. For the detailed configuration and shape of the magnetic field forming unit 27, refer to the above description of the magnetic field forming unit 130 of the plasma treatment vessel 100.

In one embodiment, the magnetic field forming unit 27 may be disposed adjacent to any one of the first electrode E1 and the second electrode E2. Alternatively, the magnetic field forming unit 27 may be disposed between an auxiliary electrode 28, which will be described below, and the second electrode E2.

In one embodiment, the magnetic field forming unit 27 may be disposed adjacent to a workpiece M accommodated in the workpiece receiving container 200.

In one embodiment, the magnetic field forming unit 27 may be disposed inside the sealing part 24. Alternatively, as shown in FIG. 17, a magnetic field forming unit 27' may be disposed on an outer surface of a sealing part 24'.

In another embodiment, the magnetic field forming unit 27" may be include two or more magnetic field forming units. Referring to FIG. 18, magnetic field forming units 27a" and 27b" may be disposed on a side surface of a sealing part 24" and above the first electrode E1, respectively. The magnetic field forming unit 27" may control the density of plasma P inside the sealing part 24" by changing the arrangement of the magnetic field forming units.

The plasma treatment device 20 according to another embodiment of the present invention may further include an auxiliary electrode 28.

The auxiliary electrode 28 may be disposed in the sealing part 24 to surround the workpiece receiving container 200. The auxiliary electrode 28 may be disposed between the first electrode E1 and the second electrode E2 and control the electric field formed inside the sealing part 24 and the density of plasma P generated by the electric field.

In one embodiment, as shown in FIGS. 16 to 18, the auxiliary electrode 28 may be disposed to extend from the first electrode E1 toward the second electrode E2. In this case, the auxiliary electrode 28 may have substantially the same potential as the first electrode E1. In this case, as shown in FIG. 16, the magnetic field forming unit 27 may be disposed adjacent to the auxiliary electrode 28 or disposed in direct contact therewith (not shown).

In one embodiment, the auxiliary electrode 28 may be a floating electrode to which no external potential is directly applied. In this case, a potential of the auxiliary electrode may be set differently according to a relative distance from the first electrode E1 and the second electrode E2.

In one embodiment, the auxiliary electrode 28 may be disposed adjacent to the workpiece M accommodated in the workpiece receiving container 200. The plasma P may be generated and concentrated in an area adjacent to the workpiece M by the auxiliary electrode 28. In this way, the plasma treatment device 20 may increase the efficiency of plasma surface treatment.

For other configurations and functions of the plasma treatment device 20, refer to the above-described content.

The plasma treatment vessel and the plasma treatment device of the present invention can uniformly perform plasma surface treatment on a workpiece while the plasma formed in the internal space rotates due to a magnetic field. In this case, by considering the physical properties and arrangement of the workpiece and the type of workpiece receiving container to adjust the configurations and arrangements of a magnetic field forming unit and an auxiliary electrode, damage to the workpiece can be minimized and the efficiency of plasma surface treatment can be improved.

As described above, while the present invention has been described with reference to the exemplary embodiments shown in the drawings, these are merely illustrative, and those skilled in the art to which the present invention pertains will be understood that various modifications and equivalent other embodiments can be implemented within the spirit and scope of the invention. Therefore, the true technical scope of the present invention should be defined by the appended claims.

Specific operations described in the embodiments are examples and do not limit the scope of the embodiments in any way. Also, unless described as "essential," "important," and the like, an element may not be absolutely necessary for the application of the present disclosure.

In the specification (especially, in the claims) of the embodiments, the use of the term "the" and similar referential terms may be used in both the singular and the plural. In addition, in the embodiments, when a range is described, the invention includes individual values that fall within the range (unless there is a description to the contrary), and it is the same as describing each individual value constituting the range in the detailed description. Finally, unless there is an explicit description of operations constituting a method according to the embodiment, the operations can be performed in any suitable order. The embodiments are not necessarily limited to the order in which the above operations are described. The use of any examples or exemplary terms (e.g., etc.) in the embodiments is merely intended to illustrate the embodiments in detail and is not intended to limit the scope of the embodiments unless otherwise defined by the appended claims. Furthermore, those skilled in the art will appreciate that various modifications, combinations, and variations can be made within the scope of the appended claims or their equivalents, according to design conditions and factors.

### [Industrial Applicability]

According to one embodiment of the present invention, a plasma treatment vessel and a plasma treatment device are provided. In addition, the embodiments of the present invention can be applied to techniques for sterilizing a workpiece or performing surface treatment on the workpiece using plasma.

## Claims

1. A plasma treatment vessel comprising:
a housing in which an internal space configured to accommodate a workpiece receiving container is formed;
a connection electrode disposed at one end of the housing, a portion of which is exposed through the internal space; and
a magnetic field forming unit disposed in the housing and configured to form a magnetic field in the internal space.

2. The plasma treatment vessel of claim 1, wherein the magnetic field forming unit is disposed symmetrically based on the internal space.

3. The plasma treatment vessel of claim 2, wherein the magnetic field forming unit includes a ring-shaped magnet surrounding the internal space.

4. The plasma treatment vessel of claim 2, wherein the magnetic field forming unit includes a plurality of magnets disposed to be spaced apart from and face each other.

5. The plasma treatment vessel of claim 1, wherein the magnetic field forming unit forms the magnetic field to allow plasma formed in the internal space to rotate based on the internal space.

6. The plasma treatment vessel of claim 1, wherein the magnetic field forming unit forms the magnetic field in a direction intersecting a direction of an electric field formed by the connection electrode.

7. The plasma treatment vessel of claim 1, wherein the magnetic field forming unit is disposed adjacent to a workpiece accommodated in the workpiece receiving container.

8. The plasma treatment vessel of claim 1, wherein the magnetic field forming unit is disposed adjacent to the connection electrode.

9. The plasma treatment vessel of claim 1, wherein the magnetic field forming unit includes two or more magnetic field forming units.

10. The plasma treatment vessel of claim 1, wherein at least a portion of the housing is made of a transparent material such that plasma treatment is checked from the outside.

11. The plasma treatment vessel of claim 1, wherein the connection electrode includes a protrusion which protrudes toward the internal space and on which the workpiece receiving container is seated.

12. The plasma treatment vessel of claim 1, further comprising an auxiliary electrode disposed in the housing to surround the workpiece receiving container and disposed adjacent to a workpiece accommodated in the workpiece receiving container.

13. The plasma treatment vessel of claim 12, wherein:
the auxiliary electrode is electrically connected to the connection electrode; and
the magnetic field forming unit is disposed in contact with the auxiliary electrode.

14. The plasma treatment vessel of claim 12, wherein the magnetic field forming unit is disposed between the auxiliary electrode and the connection electrode.

15. A plasma treatment device comprising:
a sealing part of which an interior accommodates a plasma treatment vessel, wherein the interior is sealed from an external environment;
an exhaust part configured to exhaust an atmosphere inside the sealing part and form a low-pressure atmosphere within a preset process pressure range inside the sealing part; and
a treatment part configured to form an electric field inside the sealing part and discharge the low-pressure atmosphere,
wherein the plasma treatment vessel includes:
a housing in which an internal space configured to accommodate a workpiece receiving container is formed;
a connection electrode disposed at one end of the housing, a portion of which is exposed through the internal space; and
a magnetic field forming unit disposed in the housing and configured to form a magnetic field in the internal space.

16. The plasma treatment device of claim 15, wherein the treatment part includes:
a first electrode electrically connected to the connection electrode; and
a second electrode disposed to be spaced apart from the first electrode.

17. The plasma treatment device of claim 16, wherein the plasma treatment vessel further includes a coupling magnet configured to fix the connection electrode to the first electrode.

18. The plasma treatment device of claim 15, wherein the magnetic field forming unit is disposed symmetrically based on the internal space.

19. The plasma treatment device of claim 15, wherein the magnetic field forming unit forms the magnetic field in a direction intersecting a direction of an electric field formed by the connection electrode.

20. The plasma treatment device of claim 15, wherein the magnetic field forming unit is disposed adjacent to a workpiece accommodated in the workpiece receiving container.

21. The plasma treatment device of claim 15, wherein the magnetic field forming unit is disposed adjacent to the connection electrode.

22. The plasma treatment device of claim 15, wherein the connection electrode includes a protrusion which protrudes toward the internal space and on which the workpiece receiving container is seated.

23. The plasma treatment device of claim 15, wherein the plasma treatment vessel further includes an auxiliary electrode disposed in the housing to surround the workpiece receiving container and disposed adjacent to a workpiece accommodated in the workpiece receiving container.

24. The plasma treatment device of claim 23, wherein:
the auxiliary electrode is electrically connected to the connection electrode; and
the magnetic field forming unit is disposed in contact with the auxiliary electrode.

25. The plasma treatment device of claim 23, wherein the magnetic field forming unit is disposed between the auxiliary electrode and the connection electrode.

26. A plasma treatment device comprising:
a sealing part of which an interior accommodates a workpiece receiving container, wherein the interior is sealed from an external environment;
an exhaust part configured to exhaust an atmosphere inside the sealing part and form a low-pressure atmosphere within a preset process pressure range inside the sealing part;
a treatment part configured to form an electric field inside the sealing part and discharge the low-pressure atmosphere; and
a magnetic field forming unit configured to form a magnetic field in the interior of the sealing part,
wherein the magnetic field forming unit rotates the discharged atmosphere.

27. The plasma treatment device of claim 26, wherein the treatment part includes:
a first electrode electrically connected to the workpiece receiving container; and
a second electrode disposed to be spaced apart from the first electrode.

28. The plasma treatment device of claim 27, wherein the magnetic field forming unit is disposed adjacent to one of the first electrode and the second electrode.

29. The plasma treatment device of claim 26, wherein the magnetic field forming unit is disposed symmetrically based on an internal space of the sealing part.

30. The plasma treatment device of claim 26, wherein the magnetic field forming unit is disposed adjacent to a workpiece accommodated in the workpiece receiving container.

31. The plasma treatment device of claim 27, wherein the treatment part further includes an auxiliary electrode disposed in the housing to surround the workpiece receiving container and disposed adjacent to a workpiece accommodated in the workpiece receiving container.

32. The plasma treatment device of claim 31, wherein:
the auxiliary electrode is electrically connected to the first electrode; and
the magnetic field forming unit is disposed in contact with the auxiliary electrode.

33. The plasma treatment device of claim 31, wherein the magnetic field forming unit is disposed between the auxiliary electrode and the second electrode.

34. A plasma treatment vessel comprising:
a connection electrode in which an internal space configured to accommodate a workpiece receiving container is formed; and
a magnetic field forming unit disposed in contact with the connection electrode and configured to form a magnetic field in the internal space.

35. The plasma treatment vessel of claim 34, wherein:
the magnetic field forming unit is disposed to be located inside the connection electrode; and
the connection electrode includes an opening through which the workpiece receiving container is inserted or removed.

36. The plasma treatment vessel of claim 34, wherein the connection electrode includes a protrusion which protrudes toward the internal space and on which the workpiece receiving container is seated.

37. The plasma treatment vessel of claim 34, further comprising an auxiliary electrode disposed to surround the workpiece receiving container.

38. The plasma treatment vessel of claim 36, wherein the connection electrode and the auxiliary electrode are formed integrally.
